# EUROPEAN PATENT SPECIFICATION

(11) **EP 2 696 823 B1**
(45) Date of publication and mention of the grant of the patent: **29.04.2015**
(21) Application number: 12727415.7
(22) Date of filing: 11.05.2012
(51) Int. Cl.: A61F 9/06, B23K 9/095, B23K 9/32

(54) **WELDING HELMET CONFIGURATION PROVIDING REAL-TIME FUME EXPOSURE WARNING CAPABILITY**
SCHWEISSHELMKONFIGURATION MIT FUNKTION ZUR ECHTZEIT-WARNUNG VOR RAUCHEXPOSITION
CONFIGURATION DE MASQUE DE SOUDURE FOURNISSANT UNE CAPACITÉ D'AVERTISSEMENT EN TEMPS RÉEL D'EXPOSITION À DES FUMÉES

(30) Priority: 12.05.2011 US 201113106525
(43) Date of publication of application: 19.02.2014
(73) Proprietor: Lincoln Global, Inc., City of Industry, CA 91748 (US)
(72) Inventor: DUNBAR, Douglas, N., Strongville, OH 44136 (US)
(74) Representative: Grosse Schumacher Knauer von Hirschhausen
(86) International application number: PCT/IB2012/000921
(87) International publication number: WO 2012/153184

(56) References cited:
- WO-A1-2007/029033
- WO-A1-2007/149556
- US-A1- 2002 056 458
- US-A1- 2002 184 957
- US-A1- 2008 189 820

## Description

### TECHNICAL FIELD

The present invention relates to a welding helmet.

### BACKGROUND

During a welding process (e.g., an arc welding process), contaminants such as fumes can be generated which, if breathed in my a welder, can be harmful to the welder. In many welding situations (especially indoor situations), ventilation equipment is used to draw up and vent away the fumes. However, sometimes the ventilation equipment may be inadequate for a particular welding process or scenario, or the ventilation equipment may not be properly set up or properly used by the welder. Because safety and health regulations tend to be performance based, compliance with industrial hygiene standards is dependent upon having actual workplace exposure determinations made by a qualified industrial hygienist. Current practice is to perform a full shift monitoring where a sampling device is placed on the worker to filter and collect a representative sample of the contaminants present in the worker's breathing zone. The goal is to obtain an eight hour time-weighted average concentration which may then be compared with the allowable levels in the regulations. However, many times, results cannot be obtained until several weeks after the sampling event as it is often necessary to send the samples to an accredited lab for analysis.

Further limitations and disadvantages of conventional, traditional, and proposed approaches will become apparent to one of skill in the art, through comparison of such approaches with embodiments of the present invention as set forth in the remainder of the present application with reference to the drawings. A welding helmet according to the preamble of claim 1 is known from the document WO-2007/029033.

### SUMMARY

Welding helmets, systems, and kits providing real-time fume exposure monitoring and warning capability during an arc welding process are disclosed herein. A welding helmet configured to protect the head of a user during a welding process is configured with an intelligent warning apparatus and an air-sampling pick-up and output port. The air-sampling pick-up and output port connects to a proximal end of an air sampling tube for sampling breathable air within the welding helmet, and a distal end of the air-sampling tube connects to an air-sampling in-take port of an external aerosol monitoring device. The intelligent warning apparatus communicates with the aerosol monitoring device to receive air sample output data from the aerosol monitoring device, and to process the air sample output data to generate warning data and/or warning signals based on preset exposure level set points and/or exposure warning operating modes. As a result, a welder and/or, for example, a welder's supervisor can be readily informed of any unacceptable exposure to the welder during the welding process, before any significant harm can occur to the welder. Such helmets, systems, and kits may be used as a powerful day-to-day tool for both managing and more effectively understanding workplace exposures.

These and other features of the claimed invention, as well as details of illustrated embodiments thereof, will be more fully understood from the following description, claims and drawings.

### BRIEF DESCRIPTION OF THE DRAWINGS

Fig. 1 is an illustration of a first example embodiment of a welding helmet for providing real-time fume exposure warning capability during a welding process;
Fig. 2 is an illustration of a second example embodiment of a welding helmet for providing real-time fume exposure warning capability during a welding process;
Fig. 3 is an illustration of a first embodiment of a system for providing real-time fume exposure monitoring and warning capability using the welding helmet of Fig. 1 (or, alternatively, the welding helmet of Fig. 2) during a welding process;
Fig. 4 is a functional block diagram of the system of Fig. 3 showing functional elements of a first embodiment of the intelligent warning apparatus (IWA) of the welding helmet of Fig. 1 (or the welding helmet 200 of Fig. 2);
Fig. 5 is an illustration of a system for providing real-time fume exposure monitoring and warning capability using a slightly modified embodiment of a welding helmet during a welding process; and
Fig. 6 is a functional block diagram of the system of Fig. 5 showing the functional elements of the IWA of the welding helmet of Fig. 5.

### DETAILED DESCRIPTION

Embodiments of the present invention are concerned with welding helmets, and kits providing real-time fume exposure monitoring and warning capability during an arc welding process. In accordance with certain embodiments of the present invention, such capability is provided, at least in part, in a welding helmet worn by the user performing the welding process.

As used herein, the term "integrated" refers to being positioned on, being a physically integral part of, or being attached to (with or without the capability to be subsequently unattached). As used herein, the term "real-time" refers to the monitoring, communication, and processing of air sample output data during a welding process such that a welder and/or, for example, a welder's supervisor can be readily informed of any unacceptable exposure to the welder during the welding process, before any significant harm can occur to the welder. As used herein, the term "aerosol" means a system of particles dispersed in a gas (e.g., solid fume or smoke particles dispersed in air).

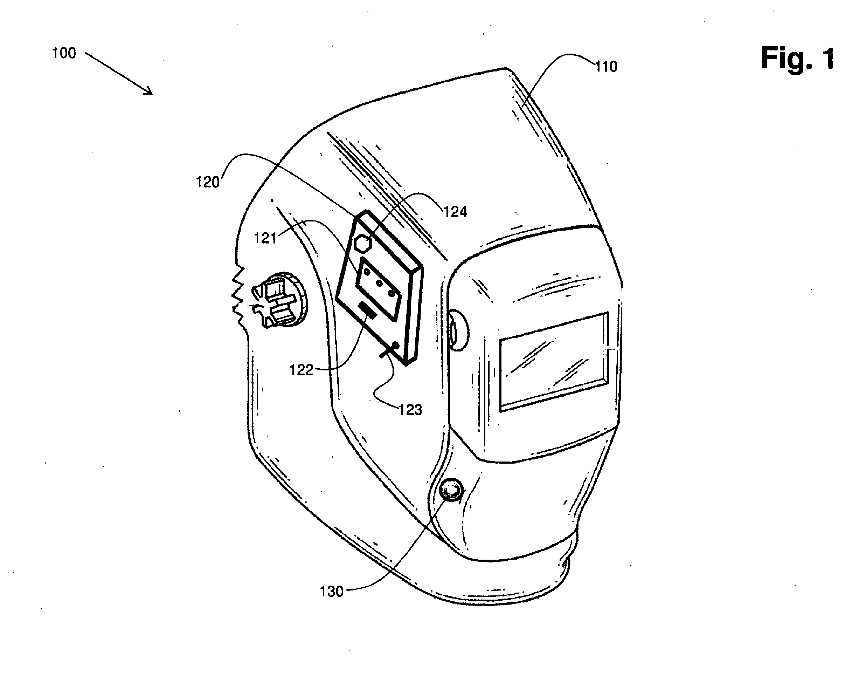

Details of various embodiments of the present invention are described below herein with respect to Figs. 1-6. Fig. 1 is an illustration of a first example embodiment of a welding helmet 100 for providing real-time fume exposure warning capability during a welding process. The welding helmet 100 includes a welding headgear 110 configured to be worn on the head of a welder to protect the welder during a welding process. The welding helmet 100 also includes an intelligent warning apparatus (IWA) 120 integrated with the welding headgear 110. The IWA 120 is configured to communicatively interface with an external aerosol monitoring (EAM) device to receive air sample output data from the EAM device. The IWA 120 generates warning information and other environmental status information in response to receiving the air sample output data from the EAM device. Details of the inter-action of the IWA 120 and the EAM device are described later herein.

In the embodiment of Fig. 1, the IWA 120 is positioned on the outside of the helmet 110. In accordance with certain embodiments of the present invention, the IWA 120 may be fixed on the welding headgear 110, or may be attachable to and detachable from the welding headgear 110. The IWA 120 includes a user interface 121, a communication input port 122 (e.g., a USB port), a radio frequency (RF) antenna 123, and an alarm or alert device 124. These elements of the IWA 120 are described in more detail later herein. In accordance with an alternative embodiment of the present invention, the alarm device 124 may be separate from the IWA 120 and integrated elsewhere on or within the helmet 100. In such an alternative embodiment, the IWA 120 would activate the alarm device 124 in a wired or wireless manner.

The welding helmet 100 further includes an air-sampling pick-up and output port (ASPOP) 130 integrated with the welding headgear 110. The ASPOP 130 is configured to sample or collect breathable air within the welding headgear 110 and to connect to a proximal end of an air sampling tube for funneling the sampled air away from the headgear 110. A distal end of the air sampling tube is attached to an EAM device as discussed later herein. As used herein, the term "breathable air" means a sample of air that is representative of the air being breathed by the welder while wearing the helmet 100. Even though the ASPOP 130 is shown near the front of the welding headgear 110 in Fig. 1, the ASPOP 130 may be located almost anywhere on the welding headgear 110 (e.g., toward the back of the welding headgear 110).

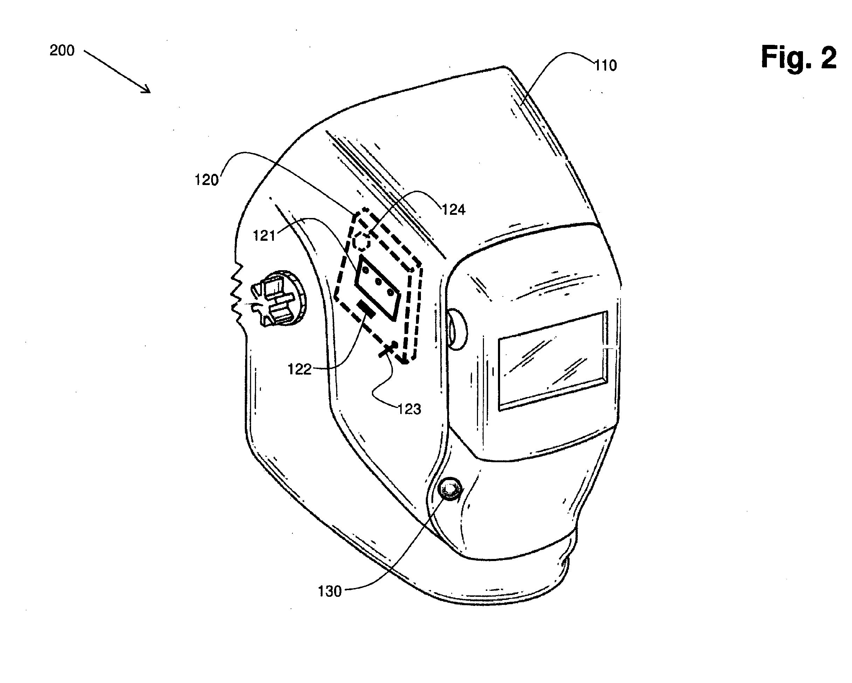

Fig. 2 is an illustration of a second example embodiment of a welding helmet 200 for providing real-time fume exposure warning capability during a welding process. The welding helmet 200 of Fig. 2 is similar to the welding helmet 100 of Fig. 1. However, the welding helmet 200 includes an IWA 120 that is largely integrated on the inside of the welding headgear 110, as indicated by the dashed lines of the IWA 120 in Fig. 2. However, the user interface 121, the communication input port 122, and the RF antenna 123 are still accessible from the outside of the welding headgear 110. The alarm or alert device 124 is on the inside of the welding headgear 110. In the embodiment of Fig. 2, much of the IWA 120 is protected by being integrated inside the welding headgear 110.

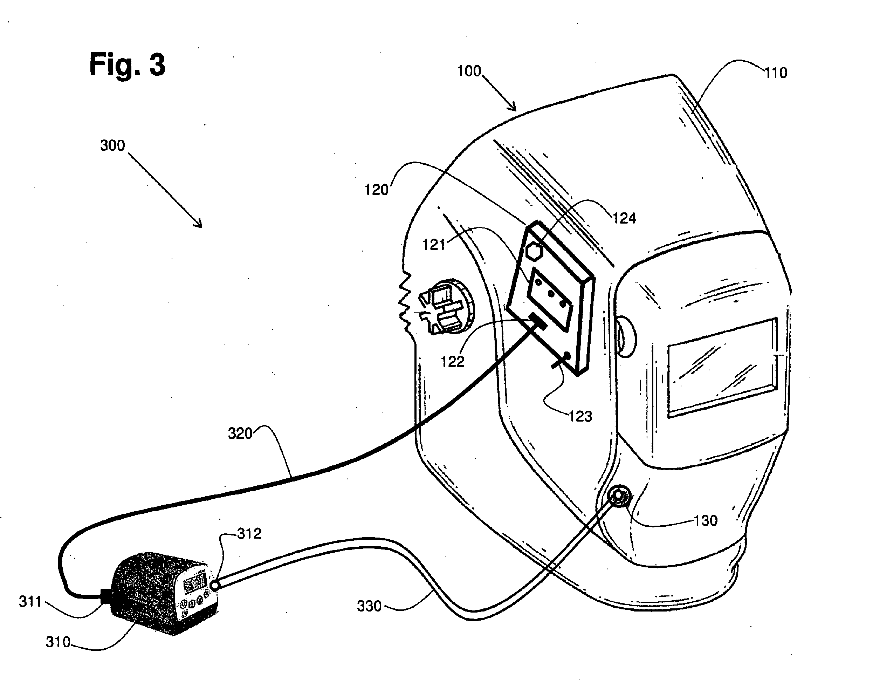

Fig. 3 is an illustration of a first embodiment of a system 300 for providing real-time fume exposure monitoring and warning capability using the welding helmet 100 of Fig. 1 (or, alternatively, the welding helmet 200 of Fig. 2) during a welding process. In addition to the welding helmet 100, the system 300 includes an external aerosol monitoring (EAM) device 310 that is external to the helmet 100. The EAM device 310 is configured to determine the concentration of one or more contaminants in the sampled breathable air and generate associated air sample output data. For example, the EAM device 310 may be a laser photometer device. Examples of such laser photometer devices are the SIDEPAK^{™} devices as manufactured by TSI, Inc. The system 300 may further include a belt or harness (not shown) for holding the EAM device 310 while the belt or harness is worn by the user.

The system 300 also includes a communication cable 320 (e.g., a USB cable) connecting the communication input port 122 of the IWA 120 with a data output port 311 of the EAM device 310. During operation, the EAM device 310 sends air sample output data to the IWA 120 via the communication cable 320. Both the EAM device 310 and the IWA 120 may be powered by a battery or a re-chargeable power pack, for example. Alternatively, the EAM device 310 and/or the IWA 120 may be powered by, for example, an AC adapter that plugs into an electrical outlet, or by an auxiliary power source of a welding power source or wire feeder.

The system 300 further includes an air-sampling tube 330. The air-sampling tube 330 may be a durable, heat-resistant, flexible plastic tube, in accordance with an embodiment of the present invention. One end of the tube 330 connects to the ASPOP 130 on the helmet 100. The other end of the tube 330 connects to the air-sampling in-take port 312 on the EAM device 310. The EAM device 310 includes a pump (not shown) to create a vacuum which draws breathable air from within the helmet 100 through the ASPOP 130, through the tube 330, through the air-sampling in-take port 312, and into the EAM device 310 for analysis. In accordance with an embodiment of the present invention, the communication cable 320 and the air-sampling tube 330 are run together as one harness to provide a "clean" design implementation. In such an embodiment, the air-sampling in-take port 312 and the data output port 311 may be relatively close together on the EAM device 310. Similarly, the communication input port 122 and the ASPOP 130 may be relatively close together on the headgear 110.

During operation of the system 300, as the EAM device 310 draws in breathable air from the helmet 100, the EAM device 310 analyzes the sampled breathable air to determine particle mass concentration (e.g., via aerosol counting) in the sampled air. In accordance with an embodiment of the present invention, the EAM device 310 is a laser photometer device capable of determining real-time aerosol mass concentration and time-weighted average (TWA) aerosol mass concentration over longer periods of time, and is capable of discriminating between various ranges of particle size.

The mass concentrations determined by the EAM device 310 are sent, via the communication cable 320, to the IWA 120 as air sample output data for processing and analysis. Other forms or types of air sample output data may be generated by the EAM 310 and sent to the IWA 120 as well, in accordance with various embodiments of the present invention. For example, the EAM device 310 may also generate and log historical exposure data over time, or even over multiple welding process sessions.

The various elements of the system 300 of Fig. 3 (welding helmet, EAM device, IWA, air-sampling tube, communication cable, and harness) may be provided in the form of a kit which can be easily and readily assembled by a user for use, and subsequently disassembled after use.

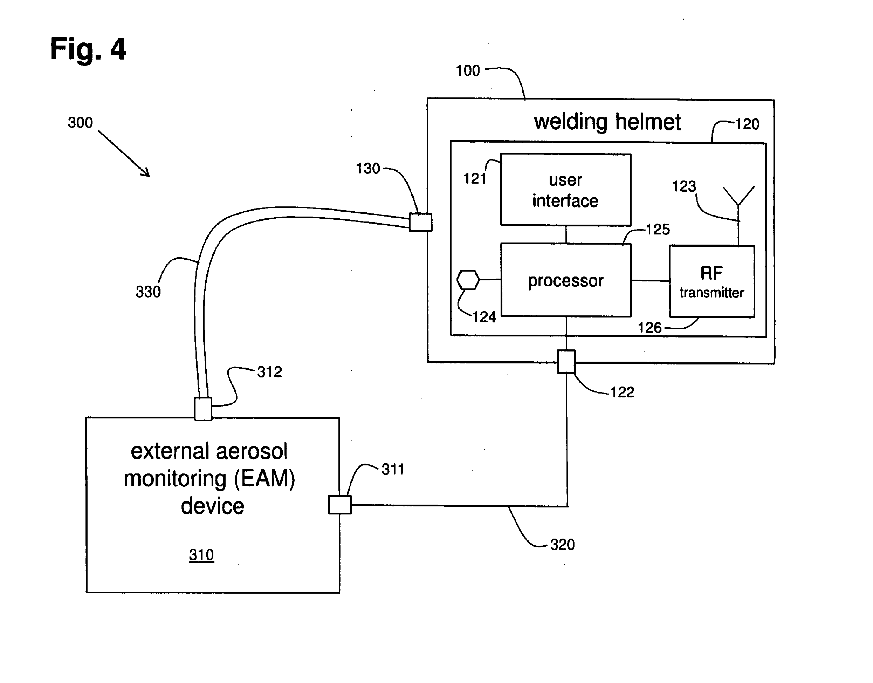

Fig. 4 is a functional block diagram of the system 300 of Fig. 3 showing functional elements of a first embodiment of the IWA 120 of the welding helmet 100 of Fig. 1 (or the welding helmet 200 of Fig. 2). As seen in Fig. 4, the IWA 120 includes a processor 125 and an RF transmitter 126 connected to the RF antenna 123. The processor 125 is operatively connected to the RF transmitter 126, the user interface 121, and the alarm or alert device 124. In accordance with various embodiments of the present invention, the processor 125 may be a software programmable microprocessor, a digital signal processor, a microcontroller, or any other processing device or chip capable of being configured or programmed to provide the processing functionality described herein for the processor 125. The processor 125 also operatively interfaces to the EAM device 310 via the communication cable 320 to receive air sample output data.

The processor 125 processes the air sample output data and generates exposure warning data and information. The exposure warning data and information may include alert data indicating when certain pre-defined exposure level limits are being exceeded, as well as other data and information including, for example, contaminant concentration level history and running time-averaged data.

The user interface 121 may be a touch-sensitive display or a configuration of switches and/or buttons, for example. Other types of user interfaces are possible as well. In one embodiment, the user interface is used by the welder to set one or more exposure level set points. An exposure level set point is a value that gets compared to the air sample output data (or a processed version thereof) by the processor 125. The air sample output data is representative of a concentration of one or more contaminants in the sampled breathable air. A user may rely on a standard maximum fume exposure guideline (MFEG) to determine an appropriate exposure level set point for a particular welding process, for example.

If the processor 125 determines that a concentration of a contaminant has been exceeded (i.e., determines that a warning condition has occurred), then the processor 124 may generate a warning signal to activate the alarm device 124 to alert the user of the situation. The alarm device 124 may be an audible alarm device, a visual alarm device, or a combination of the two, in accordance with various embodiments of the present invention. Other types of alarm devices are possible as well such as, for example, a vibrating alarm device. In accordance with an alternative embodiment of the present invention, the alarm device 124 may be separate from the helmet 100 and may be wirelessly activated by the IWA 120 of the helmet 100. Furthermore, if the welding helmet is of a type having an internal display that may be viewed by the welder, warning information from the IWA may be displayed on the internal display.

In another embodiment, the user interface is used by the welder to select a pre-defined warning operating mode. A pre-defined warning operating mode is a mode of operation of the system 300 that is programmed or configured in the processor 125 which correlates to a particular welding process and the likely contaminants that may be produced as part of the that particular welding process. A particular pre-defined warning operating mode includes preset exposure level set points and possibly other parameters and/or operating algorithms corresponding to the particular welding process. A pre-defined warning operating mode is designed to keep the welder informed (e.g., via alerts) and safe with respect to contaminant exposure during that particular welding process.

In accordance with an embodiment of the present invention, the RF transmitter 126 receives warning data and information from the processor 125 and transmits the warning data and information to an external warning station (not shown). The external warning station may be, for example, a personal computer running an exposure software application in an office just outside a welding work environment in a factory. The external warning station may be manned by a welding supervisor to keep track of exposure levels of one or more welders currently working in the welding work environment.

In accordance with another embodiment of the present invention, the RF transmitter 126 receives warning data and information from the processor 125 and transmits associated command messages or signals to a welding power source (not shown) currently being operated by a user of the system 300 to turn off or shut down the welding power source, for example, when the processor determines that contaminant levels are unsafe or are approaching unsafe levels. Similarly, the RF transmitter 126 may transmit associated command messages or signals to a ventilation system (not shown) to change an operating state of the ventilation system (e.g., to increase a fan speed of the ventilation system) if the processor determines that contaminant levels are unsafe or are approaching unsafe levels.

In accordance with other embodiments of the present invention, the RF transmitter 126 may be replaced with some other type of wireless communication device such as, for example, an infrared transmitter or a sonic transmitter. Other types of wireless communication devices may be possible as well. Accordingly, the external warning station, the welding power source, and the ventilation system would be configured to communicate with such an alternative wireless communication device.

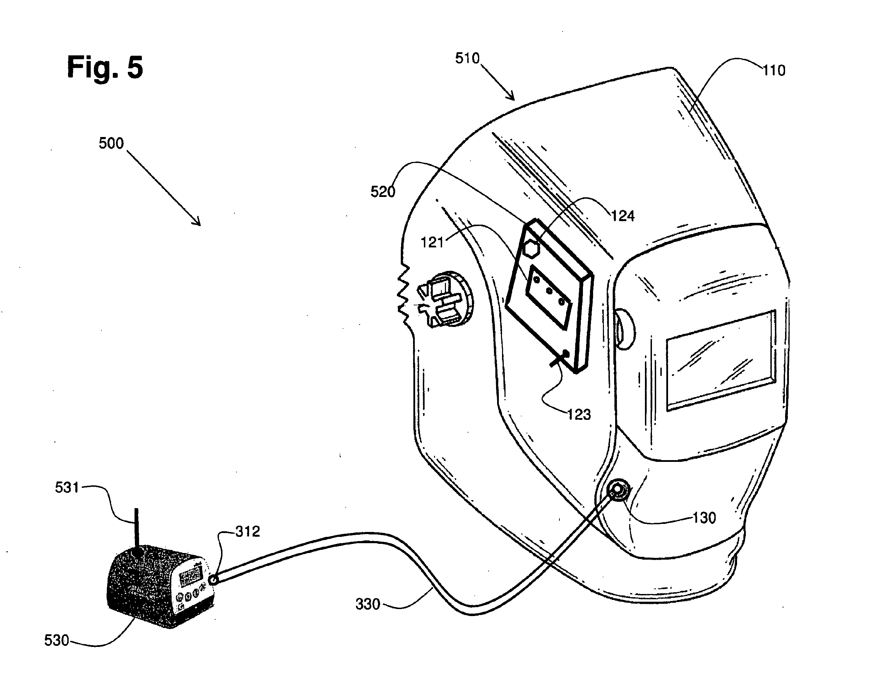

Fig. 5 is an illustration of an example of a system 500 for providing real-time fume exposure monitoring and warning capability using a slightly modified embodiment of a welding helmet 510 during a welding process. In the system 500 of Fig. 5, communication between a second embodiment of an EAM device and a second embodiment of an IWA is performed via wireless means. The EAM device 530 of Fig. 5 does not have a data output port 311 but, instead, has an RF antenna 531 for transmitting air sample output data to the IWA 520 of Fig. 5. Similarly, the IWA 520 of Fig. 5 does not have a communication input port 122 but, instead, has the RF antenna 123 for receiving air sample output data from the EAM device 530. Other wireless means of communicating air sample output data from the EAM device to the IWA may include infrared means, sonic means, or some other wireless means.

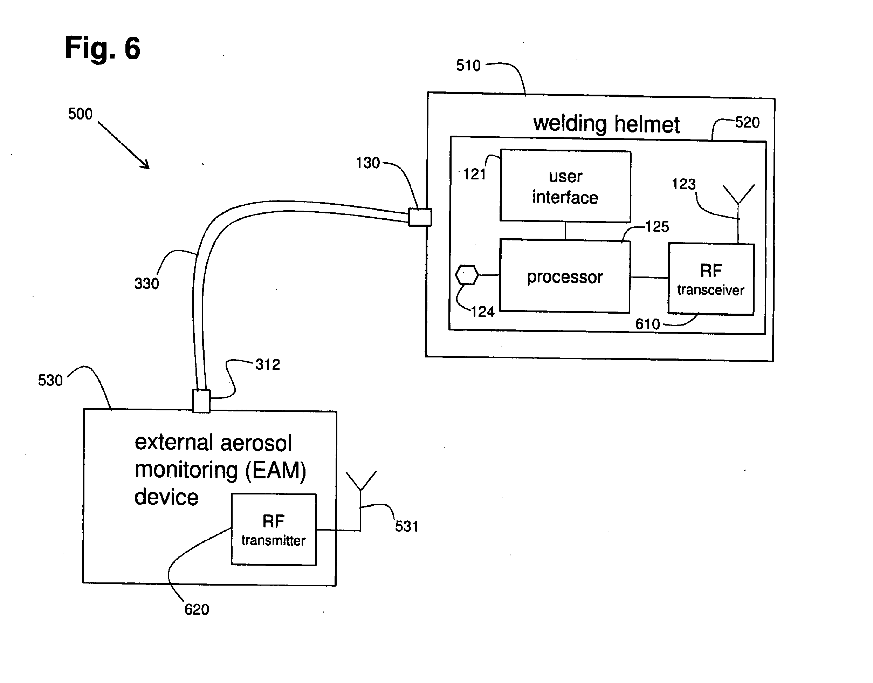

Fig. 6 is a functional block diagram of the system 500 of Fig. 5 showing the functional elements of the IWA 520 of the welding helmet 510 of Fig. 5. The EAM device 530 includes an RF transmitter 620 operatively connected to the RF antenna 531. The RF transmitter 620 functions to transmit air sample output data via the RF antenna 531 to the IWA 520. The IWA 520, instead of having an RF transmitter 126, has an RF transceiver 610 which functions to receive air sample output data via the RF antenna 123 and pass the air sample output data to the processor 125 for processing and analysis.

Also, the RF transceiver 610 functions to receive warning data and information from the processor 125 and transmit the warning data and information to an external warning station (not shown). The external warning station may be, for example, a mobile device running an exposure software application. The external warning station may be worn by an industrial hygienist inspecting a welding work environment in a factory to determine if exposure levels experienced by one or more welders currently working in the welding work environment meets current industrial standards.

Furthermore, the RF transceiver 610 may function to receive warning data and information from the processor 125 and transmit associated command messages or signals to a welding power source (not shown) currently being operated by a user of the system 500 to turn off or shut down the welding power source, for example, when the processor 125 determines that contaminant levels are unsafe or are approaching unsafe levels. Similarly, the RF transceiver 610 may transmit associated command messages or signals to a ventilation system (not shown) to change an operating state of the ventilation system (e.g., to increase a fan speed of the ventilation system) if the processor 125 determines that contaminant levels are unsafe or are approaching unsafe levels.

The RF transceiver 610 may be replaced with some other type of wireless communication device such as, for example, an infrared transceiver or a sonic transceiver. Other types of wireless communication devices may be possible as well. Accordingly, the external warning station, the welding power source, and the ventilation system would be configured to communicate with such an alternative wireless communication device.

In accordance with an embodiment of the present invention, the IWA is configured to generate and/or analyze historical exposure data. For example, the IWA may receive historical exposure data as a form of air sample output data from the EAM device and process and analyze the historical exposure data to generate statistical exposure parameters and trend data as a form of exposure warning data and information. Such statistical exposure parameters and trend data may provide great insight to an industrial hygienist with respect to the environmental operation of a plant or factory (e.g., over a full eight-hour shift, or over an entire week).

In summary, an embodiment of the present invention comprises a welding helmet providing real-time fume exposure warning capability and a kit providing real-time welding fame exposure as set forth in the appended claims.

While the claimed subject matter of the present application has been described with reference to certain embodiments, it will be understood by those skilled in the art that various changes may be made and equivalents may be substituted without departing from the scope of the claimed subject matter. Therefore, it is intended that the claimed subject matter not be limited to the particular embodiments disclosed, but that the claimed subject matter will include all embodiments falling within the scope of the appended claims.

### Reference numbers:

- 100: welding helmet
- 110: welding headgear
- 120: itelligent warning apparatus
- 121: user interface
- 122: communication input port
- 123: radio frequency antenna
- 124: alert device
- 125: processor
- 126: transmitter
- 130: output port
- 200: welding helmet
- 300: system
- 310: aerosol monitoring device
- 311: data output port
- 312: air-sampling in-take port
- 320: communication cable
- 330: air-sampling tube
- 500: system
- 510: welding helmet
- 520: IWA
- 530: EAM device
- 531: RF antenna
- 610: RF transceiver
- 620: RF transmitter

## Claims

1. A welding helmet (100) providing real-time fume exposure warning capability, said welding helmet (100) comprising:
a welding headgear (110) configured to be worn on a head of a user to protact the user during a welding process;
an intelligent warning apparatus (120) integrated with the welding headgear (110) and configured to communicatively interface with an external aerosol monitoring device (310) to receive air sample output data from said external aerosol monitoring device (310); **characterized by**
an air-sampling pick-up and output port integrated with the welding headgear (110) and configured to connect to a proximal end of an air sampling tube (330) for sampling breathable air within said welding headgear (110), wherein a distal end of said air sampling tube (330) is configured to connect to an air-sampling intake port (312) of said external aerosol monitoring device (310).

2. The welding helmet of claim 1, wherein said intelligent warning apparatus (120) includes a processor configured to process said air sample output data received from said external aerosol monitoring device (310) to generate at least warning data.

3. The welding helmet of claim 2, wherein said intelligent warning apparatus (120) further includes a user interface operatively connected to said processor and configured to allow a user to set at least one exposure level set point; and/or wherein said intelligent warning apparatus (120) further includes a user interface operatively connected to said processor and configured to allow a user to select a warning operating mode from a plurality of pre-defined warning operating modes.

4. The welding helmet of one of the claims 2 to 3, wherein said intelligent warning apparatus (120) further includes a wireless communication device operatively connected to said processor and configured to receive at least said warning data from said processor and to wirelessly transmit at least said warning data to an external warning station.

5. The welding helmet of one of the claims 2 to 5, wherein said Intelligent warning apparatus further includes an alarm device operatively connected to said processor and configured to alarm upon reception of a warning signal from said processor.

6. A kit providing real-time welding fume exposure monitoring and warning capability, said kit comprising:
a welding helmet (100) according to claim 1;
an aerosol monitoring device (310) having an air-sampling intake port (312) and configured to generate air sample output data;
an intelligent warning apparatus (120) configured to communloatively interface with said aerosol monitoring device (310) and to generate at least warning data in response to said air sample output data; and
an air-sampling tube (330) having a proximal end and a distal end, and configured to operatively mate with said air-sampling pick-up and output port of said welding helmet (100) at said proximal end of said air-sampling tube (330), and further configured to operatively mate with said air-sampling intake port (312) of said aerosol monitoring device (310) at said distal end of said air-sampling tube (330).

7. The kit of claim 7, further comprising a communication cable (320) having a proximal end and a distal end and configured to mate with a communication input port (122) of said Intelligent warning apparatus (120) at said proximal end of said communication cable (320), and further configured to mate with a data output port (311) of said aerosol monitoring device (310) at said distal end of said communication cable (320) to facilitate wired communication from said aerosol monitoring device (310) to said Intelligent warning apparatus (120).

8. The kit of one of the claims 7 to 9, wherein said intelligent warning apparatus (120) is physically Integrated with said welding helmet (100); and/orwherein said intelligent warning apparatus (120) is configured to attach to and detach from said welding helmet.

9. The kit of one of the claims 7 to 10, wherein said intelligent warning apparatus (120) Includes a wireless communication capability configured to transmit warning information generated by said intelligent warning apparatus (120) to an external warning station.

10. The kit of one of the claims 1 to 14, wherein said intelligent warning apparatus is ) further configured to communicatively interface with an external ventilation system to effect a change in an operational state of said external ventilation system in response to said warning data; and/or wherein said Intelligent warning apparatus (120) is further configured to communicatively interface with a welding power source to operatively shut down said welding power source in response to said warning data.

11. The kit of one of the claims 7 to 12, wherein said intelligent warning apparatus (120) includes a user interface configured to allow a user to set at least one exposure level set point; and/or wherein said intelligent warning apparatus (120) includes a user interface configured to allow a user to select a warning operating mode from a plurality of pre-defined warning operating modes.

12. The kit of one of the claims 7 to 13, wherein said intelligent warning apparatus (120) includes an alarm device configured to be activated when a warning condition is determined by said intelligent warning apparatus.

13. The kit of one of the claims 7 to 14, further comprising a harness for holding said aerosol monitoring device while said harness is worn by said user.

## Patentansprüche

1. Schweißerhaube (100) mit Echtzeit-Warnfähigkeit bei Kontakt mit Schweißdämpfen, wobei die Schweißerhaube (100) Folgendes umfasst:
einen Kopfaufsatz (110), der dafür konfiguriert ist, auf dem Kopf eines Benutzers getragen zu werden, um den Benutzer während eines Schweißprozesses zu schützen;
eine intelligente Warnvorrichtung (120), die in den Kopfaufsatz (110) integriert und dafür konfiguriert ist, mit einer externen Aerosolüberwachungsvorrichtung (310) zu kommunizieren, um Luftprobennahme-Ausgangsdaten von der externen Aerosolüberwachungsvorrichtung (310) zu empfangen; **gekennzeichnet durch**
einen Luft-Stichprobennahme-Aufnahme- und Ausgabeport, der in den Kopfaufsatz (110) integriert und dafür konfiguriert ist, an ein proximales Ende einer Luft-Stichprobennahmeröhre (330) angeschlossen zu werden, um Stichproben von atembarer Luft innerhalb des Kopfaufsatzes (110) zu nehmen, wobei ein distales Ende der Luft-Stichprobennahmeröhre (330) dafür konfiguriert ist, an einen LuftStichprobennahme-Einlassport (312) der externen Aerosolüberwachungsvorrichtung (310) angeschlossen zu werden.

2. Schweißerhaube nach Anspruch 1, wobei die intelligente Warnvorrichtung (120) einen Prozessor enthält, der dafür konfiguriert ist, die von der externen Aerosolüberwachungsvorrichtung (310) empfangenen Luftprobennahme-Ausgangsdaten zu verarbeiten, um mindestens Warndaten zu erzeugen.

3. Schweißerhaube nach Anspruch 2, wobei die intelligente Warnvorrichtung (120) des Weiteren eine Benutzerschnittstelle enthält, die mit dem Prozessor wirkverbunden und dafür konfiguriert ist, es einem Benutzer zu erlauben, mindestens einen Exponierungsgrad-Sollwert einzustellen; und/oder wobei die intelligente Warnvorrichtung (120) des Weiteren eine Benutzerschnittstelle enthält, die mit dem Prozessor wirkverbunden und dafür konfiguriert ist, es einem Benutzer zu erlauben, einen Warnbetriebsmodus aus mehreren vorgegebenen Warnbetriebsmodi auszuwählen.

4. Schweißerhaube nach einem der Ansprüche 2 bis 3, wobei die intelligente Warnvorrichtung (120) des Weiteren eine Drahtloskommunikationsvorrichtung enthält, die mit dem Prozessor wirkverbunden und dafür konfiguriert ist, mindestens die Warndaten von dem Prozessor zu empfangen und mindestens die Warndaten drahtlos an eine externe Warnstation zu senden.

5. Schweißerhaube nach einem der Ansprüche 2 bis 5, wobei die intelligente Warnvorrichtung des Weiteren eine Alarmvorrichtung enthält, die mit dem Prozessor wirkverbunden und dafür konfiguriert ist, bei Empfang eines Warnsignals von dem Prozessor einen Alarm auszugeben.

6. Arbeitsgerät mit Echtzeit-Überwachungs- und Warnfähigkeit bei Kontakt mit Schweißdämpfen, wobei das Arbeitsgerät Folgendes umfasst:
eine Schweißerhaube (100) nach Anspruch 1;
eine Aerosolüberwachungsvorrichtung (310), die einen Luftprobennahme-Einlassport (312) aufweist und dafür konfiguriert ist, Luftprobennahme-Ausgangedaten zu erzeugen;
eine intelligente Warnvorrichtung (120), die dafür konfiguriert ist, mit der Aerosolüberwachungsvorrichtung (310) zu kommunizieren und mindestens Warndaten in Reaktion auf die Luftprobennahme-Ausgangsdaten zu erzeugen; und
eine Luftprobennahmeröhre (330), die ein proximales Ende und ein distales Ende aufweist und dafür konfiguriert ist, funktional mit dem Luftprobennahme-Aufnahmeund Ausgabeport der Schweißerhaube (100) an dem proximalen Ende der Luft-Stichprobennahmeröhre (330) zusammenzupassen, und des Weiteren dafür konfiguriert ist, funktional mit dem Luftprobennahme-Einlassport (312) der Aerosolüberwachungsvorrichtung (310) an dem distalen Ende der Luft-Stichprobennahmeröhre (330) zusammenzupassen.

7. Arbeitsgerät nach Anspruch 7, das des Weiteren ein Kommunikationskabel (320) umfasst, das ein proximales Ende und ein distales Ende aufweist und dafür konfiguriert ist, mit einem Kommunikationseingangsport (122) der intelligenten Warnvorrichtung (120) am dem proximalen Ende des Kommunikationskabels (320) zusammenzupassen, und des Weiteren dafür konfiguriert ist, mit einem Datenausgangsport (311) der Aerosolüberwachungsvorrichtung (310) an dem distalen Ende des Kommunikationskabels (320) zusammenzupassen, um eine drahtgebundene Kommunikation von der Aerosolüberwachungsvorrichtung (310) zu der intelligenten Warnvorrichtung (120) zu ermöglichen.

8. Arbeitsgerät nach einem der Ansprüche 7 bis 9, wobei die intelligente Warnvorrichtung (120) physisch in die Schweißerhaube (100) integriert ist, und/oder wobei die intelligente Warnvorrichtung (120) so konfiguriert ist, dass sie an die Schweißerhaube angeschlossen und von der Schweißerhaube getrennt werden kann.

9. Arbeitsgerät nach einem der Ansprüche 7 bis 10, wobei die intelligente Warnvorrichtung (120) eine Drahtloskommunikationsfähigkeit enthält, die dafür konfiguriert ist, Warninformation, die durch die Intelligent Warnvorrichtung (120) erzeugt werden, an eine externe Warnstation zu senden.

10. Arbeitsgerät nach einem der Ansprüche 1 bis 14, wobei die intelligente Warnvorrichtung des Weiteren dafür konfiguriert ist, mit einem externen Belüftungssystem zu kommunizieren, um einen Wechsel eines Betriebszustandes des externen Belüttungssystem in Reaktion auf die Warndaten herbeizuführen; und/oder wobei die intelligente Warnvorrichtung (120) des Weiteren dafür konfiguriert ist, mit einer Schweißstromquelle zu kommunizieren, um die Schweißstromquelle in Reaktion auf die Warndaten abzuschalten.

11. Arbeitsgerät nach einem der Ansprüche 7 bis 12, wobei die intelligente Warnvorrichtung (120) eine Benutzerschnittstelle enthält, die dafür konfiguriert ist, es einem Benutzer zu erlauben, mindestens einen Exponierungsgrad-Sollwert einzustellen; und/oder wobei die intelligente Warnvorrichtung (120) eine Benutzerschnittstelle enthält, die dafür konfiguriert ist, es einem Benutzer zu erlauben, einen Warnbetriebsmodus aus mehreren vorgegebenen Warnbetriebsmodi auszuwählen.

12. Arbeitsgerät nach einem der Ansprüche 7 bis 13, wobei die intelligente Warnvorrichtung (120) eine Alarmvorrichtung enthält, die dafür konfiguriert ist, aktiviert zu werden, wenn ein Warnzustand durch die intelligente Warnvorrichtung festgestellt wird.

13. Arbeitsgerät nach einem der Ansprüche 7 bis 14, die des Weiteren ein Geschirr zum Halten der Aerosolüberwachungsvorrichtung umfasst, während das Geschirr durch den Benutzer getragen wird.

## Revendications

1. Masque de soudure (100) fournissant une capacité d'avertissement en temps réel d'exposition à des fumées, ledit masque de soudure (100) comprenant :
un casque de soudure (110) configuré pour être porté sur une tête d'un utilisateur pour protéger l'utilisateur au cours d'un processus de soudure ;
un appareil d'avertissement intelligent (120) intégré au casque de soudure (110) et configuré pour être en interface de manière à pouvoir communiquer avec un dispositif de surveillance d'aérosol externe (310) pour recevoir des données de sortie d'échantillon d'air dudit dispositif de surveillance d'aérosol externe (310) ; **caractérisé par**
un orifice de prise d'échantillonnage d'air et de sortie intégré au casque de soudure (110) et configuré pour se relier à une extrémité proximale d'un tube d'échantillonnage d'air (330) pour échantillonner de l'air respirable à l'intérieur dudit casque de soudure (110), dans lequel une extrémité distale dudit tube d'échantillonnage d'air (330) est configurée pour se relier à un orifice d'admission d'échantillonnage d'air (312) dudit dispositif de surveillance d'aérosol externe (310).

2. Masque de soudure selon la revendication 1, dans lequel ledit appareil d'avertissement intelligent (120) comprend un processeur configuré pour traiter lesdites données de sortie d'échantillon d'air reçues dudit dispositif de surveillance d'aérosol externe (310) pour générer au moins des données d'avertissement.

3. Masque de soudure selon la revendication 2, dans lequel ledit appareil d'avertissement intelligent (120) comprend en outre une interface utilisateur reliée de manière opérationnelle au dit processeur et configurée pour permettre à un utilisateur de régler au moins un point de consigne de niveau d'exposition ; et/ou dans lequel ledit appareil d'avertissement intelligent (120) comprend en outre une interface utilisateur reliée de manière opérationnelle au dit processeur et configurée pour permettre à un utilisateur de sélectionner un mode de fonctionnement d'avertissement parmi une pluralité de modes de fonctionnement d'avertissement prédéfinis.

4. Masque de soudure selon la revendication 2 ou 3, dans lequel ledit appareil d'avertissement intelligent (120) comprend en outre un dispositif de communication sans fil relié de manière opérationnelle au dit processeur et configuré pour recevoir au moins lesdites données d'avertissement provenant dudit processeur et transmettre sans fil au moins lesdites données d'avertissement à une station d'avertissement externe.

5. Masque de soudure selon l'une des revendications 2 à 5, dans lequel ledit appareil d'avertissement intelligent comprend en outre un dispositif d'alarme relié de manière opérationnelle au dit processeur et configuré pour déclencher une alarme à la réception d'un signal d'avertissement provenant dudit processeur.

6. Kit fournissant une capacité de surveillance et d'avertissement d'exposition en temps réel à des fumées de soudure, ledit kit comprenant :
un masque de soudure (100) selon la revendication 1 ;
un dispositif de surveillance d'aérosol (310) comportant un orifice d'admission d'échantillonnage d'air (312) et configuré pour générer des données de sortie d'échantillon d'air ;
un appareil d'avertissement intelligent (120) configuré pour être en interface de manière à pouvoir communiquer avec ledit dispositif de surveillance d'aérosol (310) et pour générer au moins des données d'avertissement en réponse aux dites données de sortie d'échantillon d'air ; et
un tube d'échantillonnage d'air (330) comportant une extrémité proximale et une extrémité distale, et configuré pour s'accoupler de manière opérationnelle au dit orifice de prise d'échantillonnage d'air et de sortie dudit masque de soudure (100) à ladite extrémité proximale dudit tube d'échantillonnage d'air (330), et en outre configuré pour s'accoupler de manière opérationnelle au dit orifice d'admission d'échantillonnage d'air (312) dudit dispositif de surveillance d'aérosol (310) à ladite extrémité distale dudit tube d'échantillonnage d'air (330).

7. Kit selon la revendication 7, comprenant en outre un câble de communication (320) ayant une extrémité proximale et une extrémité distale et configuré pour s'accoupler à un orifice d'entrée de communication (122) dudit appareil d'avertissement intelligent (120) à ladite extrémité proximale dudit câble de communication (320), et en outre configuré pour s'accoupler à un orifice de sortie de données (311) dudit dispositif de surveillance d'aérosol (310) à ladite extrémité distale dudit câble de communication (320) pour faciliter une communication filaire dudit dispositif de surveillance d'aérosol (310) au dit appareil d'avertissement intelligent (120).

8. Kit selon la revendication 6 à 9, dans lequel ledit appareil d'avertissement intelligent (120) est physiquement intégré au dit masque de soudure (100) ; et/ou dans lequel ledit appareil d'avertissement intelligent (120) est configuré pour s'attacher au dit masque de soudure et s'en détacher.

9. Kit selon l'une des revendications 7 à 10, dans lequel ledit appareil d'avertissement intelligent (120) comprend une capacité de communication sans fil configurée pour transmettre des informations d'avertissement générées par ledit appareil d'avertissement intelligent (120) à une station d'avertissement externe.

10. Kit selon l'une des revendications 1 à 14, dans lequel ledit appareil d'avertissement intelligent est en outre configuré pour être en interface de manière à pouvoir communiquer avec un système de ventilation externe afin d'effectuer un changement d'un état opérationnel dudit système de ventilation externe en réponse aux dites données d'avertissement ; et/ou dans lequel ledit appareil d'avertissement Intelligent (120) est en outre configuré pour être en interface de manière à pouvoir communiquer avec une source d'alimentation électrique de soudure pour arrêter de manière opérationnelle ladite source d'alimentation électrique de soudure en réponse aux dites données d'avertissement.

11. Kit selon l'une des revendications 7 à 12, dans lequel ledit appareil d'avertissement intelligent (120) comprend une interface utilisateur configurée pour permettre à un utilisateur de régler au moins un point de consigne de niveau d'exposition ; et/ou dans lequel ledit appareil d'avertissement intelligent (120) comprend une interface utilisateur configurée pour permettre à un utilisateur de sélectionner un mode de fonctionnement d'avertissement parmi une pluralité de modes de fonctionnement d'avertissement prédéfinis.

12. Kit selon l'une des revendications 7 à 13, dans lequel ledit appareil d'avertissement intelligent (120) comprend un dispositif d'alarme configuré pour être activé lorsqu'une condition d'avertissement est déterminée par ledit appareil d'avertissement intelligent.

13. Kit selon l'une des revendications 7 à 14, comprenant en outre un harnais pour maintenir ledit dispositif de surveillance d'aérosol pendant que ledit harnais est porté par ledit utilisateur.
